# EUROPEAN PATENT SPECIFICATION

(11) **EP 2 335 782 B1**
(45) Date of publication and mention of the grant of the patent: **05.08.2020**
(21) Application number: 10014872.5
(22) Date of filing: 26.06.2006
(51) Int. Cl.: A62D 1/00

(54) **Use of compositions comprising HFCO-1233zd**
Verwendung von Zusammensetzungen die HFCO-1233zd enthalten
Utilisations des compositions comprenant du HFCO-1233zd

(30) Priority: 24.06.2005 US 693853 P
(43) Date of publication of application: 22.06.2011
(62) Divisional of application: 06785612.0
(73) Proprietor: Honeywell International Inc., Morris Plains, NJ 07950 (US)
(72) Inventor: Singh, Rajiv R., Morristown, NJ 07962-2245 (US); Pham, Hang T., Morristown, NJ 07962-2245 (US)
(74) Representative: Crooks, Elizabeth Caroline

(56) References cited:
- WO-A2-2004/037913
- US-A- 5 449 041
- US-A- 5 710 352
- US-A1- 2001 032 960
- J Douglas Mather ET AL: "PROJECT REVIEWS: TROPODEGRADABLE BROMOCARBON EXTINGUISHANTS -11 AND PLUOROALKYL PHOSPHORUS COMPOUNDS", , 1 January 2001 (2001-01-01), XP055106093, Retrieved from the Internet: URL:http://fire.nist.gov/bfrlpubs/fire02/P DF/f02173.pdf [retrieved on 2014-03-07]
- DATABASE WPI Week 199221 Thomson Scientific, London, GB; AN 1992-172539 XP002759309, -& JP H04 110388 A (DAIKIN KOGYO KK) 10 April 1992 (1992-04-10)

## Description

### FIELD OF THE INVENTION

This invention relates to use of a composition comprising 1,1,1-trifluoro-3-chloropropene (HFCO-1233zd) in a method of suppressing a flame, said method comprising contacting said flame with said composition. This invention further relates to a method of reducing the flammability of a fluid, the method comprising the step of adding a composition comprising HFCO-1233zd to said fluid.

### BACKGROUND

Fluorocarbon based fluids have found widespread use in many commercial and industrial applications, including as the working fluid in systems such as air conditioning, heat pump and refrigeration systems, as aerosol propellants, as blowing agents, as heat transfer media, and as gaseous dielectrics. Because of certain suspected environmental problems, including the relatively high global warming potentials, associated with the use of some of the compositions that have heretofore been used in these applications, it has become increasingly desirable to use fluids having low or even zero ozone depletion potential, such as hydrofluorocarbons ("HFCs"). Thus, the use of fluids that do not contain chlorofluorocarbons ("CFCs") or hydrochlorofluorocarbons ("HCFCs") is desirable. Furthermore, some HFC fluids may have relatively high global warming potentials associated therewith, and it is desirable to use hydrofluorocarbon or other fluorinated fluids having as low global warming potentials as possible while maintaining the desired performance in use properties. Additionally, the use of single component fluids or azeotrope-like mixtures, which do not substantially fractionate on boiling and evaporation, is desirable in certain circumstances.

Certain fluorocarbons have been a preferred component in many heat exchange fluids, such as refrigerants, for many years in many applications. For, example, fluoroalkanes, such as chlorofluoromethane and chlorofluoroethane derivatives, have gained widespread use as refrigerants in applications including air conditioning and heat pump applications owing to their unique combination of chemical and physical properties. Many of the refrigerants commonly utilized in vapor compression systems are either single components fluids or azeotropic mixtures.

As suggested above, concern has been increasing in recent years about potential damage to the earth's atmosphere and climate, and certain chlorine-based compounds have been identified as particularly problematic in this regard. The use of chlorine-containing compositions (such as chlorofluorocarbons (CFC's), hydrochlorofluorocarbons (HCF's) and the like) as the working fluid in heat transfer systems, such as in refrigeration and air-conditioning systems, has become disfavored because of the ozone-depleting properties associated with many of such compounds. There has thus been an increasing need for new fluorocarbon and hydrofluorocarbon compounds and compositions that are attractive alternatives to the compositions heretofore used in these and other applications. For example, it has become desirable to retrofit chlorine-containing refrigeration systems by replacing chlorine-containing refrigerants with non-chlorine-containing refrigerant compounds that will not deplete the ozone layer, such as hydrofluorocarbons (HFC's). Industry in general and the heat transfer industry in particular are continually seeking new fluorocarbon based mixtures that offer alternatives to, and are considered environmentally safer substitutes for, CFCs and HCFCs. It is generally considered important, however, at least with respect to heat transfer fluids, that any potential substitute must also possess those properties present in many of the most widely used fluids, such as excellent heat transfer properties, chemical stability, low- or no- toxicity, non-flammability and/or lubricant compatibility, among others.

Applicants have come to appreciate that lubricant compatibility is of particular importance in many of applications. More particularly, it is highly desirable for refrigeration fluids to be compatible with the lubricant utilized in the compressor unit, used in most refrigeration systems. Unfortunately, many non-chlorine-containing refrigeration fluids, including HFC's, are relatively insoluble and/or immiscible in the types of lubricants used traditionally with CFC's and HFC's, including, for example, mineral oils, alkylbenzenes or poly(alpha-olefins). In order for a refrigeration fluid-lubricant combination to work at a desirable level of efficiently within a compression refrigeration, air-conditioning and/or heat pump system, the lubricant should be sufficiently soluble in the refrigeration liquid over a wide range of operating temperatures. Such solubility lowers the viscosity of the lubricant and allows it to flow more easily throughout the system. In the absence of such solubility, lubricants tend to become lodged in the coils of the evaporator of the refrigeration, air-conditioning or heat pump system, as well as other parts of the system, and thus reduce the system efficiency.

With regard to efficiency in use, it is important to note that a loss in refrigerant thermodynamic performance or energy efficiency may have secondary environmental impacts through increased fossil fuel usage arising from an increased demand for electrical energy.

Furthermore, it is generally considered desirably for CFC refrigerant substitutes to be effective without major engineering changes to conventional vapor compression technology currently used with CFC refrigerants.

Flammability is another important property for many applications. That is, it is considered either important or essential in many applications, including particularly in heat transfer applications, to use compositions which are non-flammable. Thus, it is frequently beneficial to use in such compositions compounds which are nonflammable. As used herein, the term "nonflammable" refers to compounds or compositions which are determined to be nonflammable as determined in accordance with ASTM standard E-681, dated 2002, which is incorporated herein by reference. Unfortunately, many HFC's which might otherwise be desirable for used in refrigerant compositions are not nonflammable. For example, the fluoroalkane difluoroethane (HFC-152a) and the fluoroalkene 1,1,1-trifluoropropene (HFO-1243zf) are each flammable and therefore not viable for use in many applications.

Higher fluoroalkenes, that is fluorine-substituted alkenes having at least five carbon atoms, have been suggested for use as refrigerants. U.S. Patent No. 4,788,352 - Smutny is directed to production of fluorinated C₅ to C₈ compounds having at least some degree of unsaturation. The Smutny patent identifies such higher olefins as being known to have utility as refrigerants, pesticides, dielectric fluids, heat transfer fluids, solvents, and intermediates in various chemical reactions. (See column 1, lines 11-22).

While the fluorinated olefins described in Smutny may have some level of effectiveness in heat transfer applications, it is believed that such compounds may also have certain disadvantages. For example, some of these compounds may tend to attack substrates, particularly general-purpose plastics such as acrylic resins and ABS resins. Furthermore, the higher olefinic compounds described in Smutny may also be undesirable in certain applications because of the potential level of toxicity of such compounds which may arise as a result of pesticide activity noted in Smutny. Also, such compounds may have a boiling point which is too high to make them useful as a refrigerant in certain applications.

Bromofluoromethane and bromochlorofluoromethane derivatives, particularly bromotrifluoromethane (Halon 1301) and bromochlorodifluoromethane (Halon 1211) have gained widespread use as fire extinguishing agents in enclosed areas such as airplane cabins and computer rooms. However, the use of various halons is being phased out due to their high ozone depletion. Moreover, as halons are frequently used in areas where humans are present, suitable replacements must also be safe to humans at concentrations necessary to suppress or extinguish fire.

WO2004/037913 discloses the use of HFO-1225 and HFO-1234 in refrigeration equipment.

US 5,710,352 discloses a method for the preparation of HFC-245fa and HCFC-1233.

Applicants have thus come to appreciate a need for compositions, and particularly fire extinguishing/suppression compositions, that are potentially useful in numerous applications, while avoiding one or more of the disadvantages noted above.

### SUMMARY

Applicants have found that the above-noted need, and other needs, can be satisfied by the use of compositions comprising 1,1,1-trifluoro-3-chloropropene (HFCO-1233zd).

The term HFCO-1233zd is used herein generically to refer to 1,1,1-trifluoro-3,chloro-propene, independent of whether it is the cis- or trans- form. The terms "cisHFCO- 1233zd" and "transHFCO-1233zd" are used herein to describe the cis- and trans- forms of 1, 1, 1-trifluoro,3-chlororopropene, respectively. The term "HFCO-1233zd" therefore includes within its scope cisHFCO-1233zd, transHFCO-1233zd, and all combinations and mixtures of these.

### DETAILED DESCRIPTION

### THE COMPOSITIONS

Described herein are compositions comprising 1,1,1-trifluoro-3-chloropropene (HFCO-1233zd).

The compositions described herein are believed to possess properties that are advantageous for a number of important reasons. For example, applicants believe, based at least in part on mathematical modeling, that the fluoroolefins described herein will not have a substantial negative affect on atmospheric chemistry, being negligible contributors to ozone depletion in comparison to some other halogenated species. The compositions described herein thus have the advantage of not contributing substantially to ozone depletion. The compositions also do not contribute substantially to global warming compared to many of the hydrofluoroalkanes presently in use.

Of course other compounds and/or components that modulate a particular property of the compositions (such as cost for example) may also be included in the compositions, and the presence of all such compounds and components is contemplated herein.

In certain preferred forms, compositions described herein have a Global Warming Potential (GWP) of not greater than about 1000, more preferably not greater than about 500, and even more preferably not greater than about 150. The GWP of the compositions described herein may not be greater than about 100 and even more preferably not greater than about 75. As used herein, "GWP" is measured relative to that of carbon dioxide and over a 100 year time horizon, as defined in "The Scientific Assessment of Ozone Depletion, 2002, a report of the World Meteorological Association's Global Ozone Research and Monitoring Project".

In certain preferred forms, the compositions also preferably have an Ozone Depletion Potential (ODP) of not greater than 0.05, more preferably not greater than 0.02 and even more preferably about zero. As used herein, "ODP" is as defined in "The Scientific Assessment of Ozone Depletion, 2002, A report of the World Meteorological Association's Global Ozone Research and Monitoring Project".

The amount of the 1,1,1-trifluoro-3-chloropropene (HFCO-1233zd), can vary widely, depending the particular application, and compositions containing more than trace amounts and less than 100% of the compound are contemplated herein. Moreover, the compositions can be azeotropic, azeotrope-like or non-azeotropic. Preferably, the compositions described herein comprise 1,1,1-trifluoro-3-chloropropene (HFCO-1233zd), in amounts from about 5% by weight to about 99% by weight, and even more preferably from about 5% to about 95%. Many additional compounds or components, including lubricants, stabilizers, metal passivators, corrosion inhibitors, flammability suppressants, and other compounds and/or components that modulate a particular property of the compositions (such as cost for example) may be included in the compositions described herein, and the presence of all such compounds and components is contemplated herein. The compositions may include, in addition to the 1,1,1-trifluoro-3-chloropropene (HFCO-1233zd), one or more of the following:
Trichlorofluoromethane (CFC-11)
Dichlorodifluoromethane (CFC-12)
Difluoromethane (HFC-32)
Pentafluoroethane (HFC-125)
1,1,2,2-tetrafluoroethane (HFC-134) 1,1,1,2-Tetrafluoroethane (HFC-134a) Difluoroethane
(HFC-152a)
1,1,1,2,3,3,3-Heptafluoropropane (HFC-227ea)
1,1,1,3,3,3-hexafluoropropane (HFC-236fa)
1,1,1,3,3-pentafluoropropane (HFC-245fa)
1,1,1,3,3-pentafluorobutane (HFC-365mfc)
water
CO₂

The relative amount of any of the above noted compounds, as well as any additional components which may be included in the compositions, can vary widely according to the particular application for the composition, and all such relative amounts are considered to be within the scope hereof.

The compositions described herein are generally useful as replacements for CFCs, such as dichlorodifluoromethane (CFC-12), HCFCs, such as chlorodifluoromethane (HCFC22), HFCs, such as tetrafluoroethane (HFC-134a), and combinations of HFCs and CFCs, such as the combination of CFC-12 and 1,1-difluorethane (HFC-152a) (the combination CFC-12:HFC-152a in a 73.8:26.2 mass ratio being known as R-500) in refrigerant, aerosol, and other applications.

### METHODS AND SYSTEMS

Methods of extinguishing and suppressing fire are described, and methods of reducing the flammability of a fluid.

### FLAMMABILITY REDUCTION METHODS

The present invention provides methods for reducing the flammability of fluids, said methods comprising adding a compound or composition described herein to said fluid. The flammability associated with any of a wide range of otherwise flammable fluids may be reduced according to the present invention. For example, the flammability associated with fluids such as ethylene oxide, flammable hydrofluorocarbons and hydrocarbons, including: HFC-152a, 1,1,1-trifluoroethane (HFC-143a), difluoromethane (HFC-32), propane, hexane, octane, and the like can be reduced according to the present invention. For the purposes of the present invention, a flammable fluid may be any fluid exhibiting flammability ranges in air as measured via any standard conventional test method, such as ASTM E-681, and the like.

Any suitable amounts of the compounds or compositions described herein may be added to reduce flammability of a fluid according to the present invention. As will be recognized by those of skill in the art, the amount added will depend, at least in part, on the degree to which the subject fluid is flammable and the degree to which it is desired to reduce the flammability thereof. In certain preferred embodiments, the amount of compound or composition added to the flammable fluid is effective to render the resulting fluid substantially non-flammable.

### FLAME SUPPRESSION METHODS

The present invention further provides methods of suppressing a flame, said methods comprising contacting a flame with a fluid comprising a compound or composition described herein. Any suitable methods for contacting the flame with the composition may be used. For example, a composition may be sprayed, poured, and the like onto the flame, or at least a portion of the flame may be immersed in the composition. In light of the teachings herein, those of skill in the art will be readily able to adapt a variety of conventional apparatus and methods of flame suppression for use in the present invention.

## Claims

1. Use of a composition comprising 1,1,1 -trifluoro-3-chloropropene (HFCO-1233zd), in a method of suppressing a flame, said method comprising contacting said flame with said composition.

2. Use according to claim 1, wherein the HCFO-1233zd is cis HFCO-1233zd, trans HFCO-1233zd or a combination thereof, preferably trans HFCO-1233zd.

3. Use according to any preceding claim, wherein the composition has a Global Warming Potential (GWP) of not greater than 1000, preferably not greater than 500, more preferably not greater than 150, more preferably not greater than 100, more preferably not greater than 75.

4. Use according to any preceding claim, wherein the composition comprises from about 5% to about 99 %, and preferably from about 5 % to about 95 %, by weight of the HFCO-1233zd.

5. Use according to any preceding claim, wherein the composition is sprayed or poured onto the flame, or wherein at least a portion of the flame is immersed in the composition.

6. A method of reducing the flammability of a fluid, the method comprising the step of adding a composition comprising HFCO-1233zd to said fluid.

7. The method of claim 6, wherein the HCFO-1233zd is cis HFCO-1233zd, trans HFCO-1233zd or a combination thereof, preferably trans HFCO-1233zd.

8. The method of claims 6 or 7, wherein the composition has a Global Warming Potential (GWP) of not greater than 1000, preferably not greater than 500, more preferably not greater than 150, more preferably not greater than 100, more preferably not greater than 75.

9. The method of claims 6 to 8, wherein the composition comprises from about 5% to about 99 %, and preferably from about 5 % to about 95 %, by weight of the HFCO-1233zd.

## Patentansprüche

1. Verwendung einer Zusammensetzung, die 1,1,1-Trifluor-3-chlorpropen (HFCO-1233zd) umfasst, bei einem Verfahren zur Unterdrückung einer Flamme, wobei das Verfahren das Inkontaktbringen der Flamme mit der Zusammensetzung umfasst.

2. Verwendung nach Anspruch 1, wobei es sich bei dem HFCO-1233zd um cis-HFCO-1233zd, trans-HFCO-1233zd oder eine Kombination davon, vorzugsweise trans-HFCO-1233zd, handelt.

3. Verwendung nach einem der vorhergehenden Ansprüche, wobei die Zusammensetzung ein Treibhauspotential (Global Warming Potential, GWP) von nicht mehr als 1000, vorzugsweise nicht mehr als 500, weiter bevorzugt nicht mehr als 150, weiter bevorzugt nicht mehr als 100, weiter bevorzugt nicht mehr als 75, aufweist.

4. Verwendung nach einem der vorhergehenden Ansprüche, wobei die Zusammensetzung etwa 5 bis etwa 99 Gew.-% und vorzugsweise etwa 5 bis etwa 95 Gew.-% des HFCO-1233zd umfasst.

5. Verwendung nach einem der vorhergehenden Ansprüche, wobei die Zusammensetzung auf die Flamme gesprüht oder gegossen wird oder wobei mindestens ein Teil der Flamme in die Zusammensetzung eingetaucht wird.

6. Verfahren zur Verringerung der Entflammbarkeit eines Fluids, bei dem man eine Zusammensetzung, die HFCO-1233zd umfasst, zu dem Fluid gibt.

7. Verfahren nach Anspruch 6, wobei es sich bei dem HFCO-1233zd um cis-HFCO-1233zd, trans-HFCO-1233zd oder eine Kombination davon, vorzugsweise trans-HFCO-1233zd, handelt.

8. Verfahren nach Anspruch 6 oder 7, wobei die Zusammensetzung ein Treibhauspotential (Global Warming Potential, GWP) von nicht mehr als 1000, vorzugsweise nicht mehr als 500, weiter bevorzugt nicht mehr als 150, weiter bevorzugt nicht mehr als 100, weiter bevorzugt nicht mehr als 75, aufweist.

9. Verfahren nach den Ansprüchen 6 bis 8, wobei die Zusammensetzung etwa 5 bis etwa 99 Gew.-% und vorzugsweise etwa 5 bis etwa 95 Gew.-% des HFCO-1233zd umfasst.

## Revendications

1. Utilisation d'une composition comprenant du 1,1,1-trifluoro-3-chloropropène (HFCO-1233zd), dans un procédé de suppression d'une flamme, ledit procédé comprenant la mise en contact de ladite flamme avec ladite composition.

2. Utilisation selon la revendication 1, le HFCO-1233zd étant le cis-HFCO-1233zd, le trans-HFCO-1233zd ou une combinaison correspondante, préférablement le trans-HFCO-1233zd.

3. Utilisation selon une quelconque revendication précédente, la composition possédant un Pouvoir de Réchauffement Global (PGR) non supérieur à 1 000, préférablement non supérieur à 500, plus préférablement non supérieur à 150, plus préférablement non supérieur à 100, plus préférablement non supérieur à 75.

4. Utilisation selon une quelconque revendication précédente, la composition comprenant d'environ 5 % à environ 99 %, et préférablement d'environ 5 % à environ 95 % en poids du HFCO-1233zd.

5. Utilisation selon une quelconque revendication précédente, la composition étant pulvérisée ou versée sur la flamme, ou au moins une partie de la flamme étant immergée dans la composition.

6. Procédé de réduction de l'inflammabilité d'un fluide, le procédé comprenant l'étape d'ajout d'une composition comprenant du HFCO-1233zd audit fluide.

7. Procédé selon la revendication 6, le HFCO-1233zd étant le cis-HFCO-1233zd, le trans-HFCO-1233zd ou une combinaison correspondante, préférablement le trans-HFCO-1233zd.

8. Procédé selon la revendication 6 ou 7, la composition possédant un Pouvoir de Réchauffement Global (PGR) non supérieur à 1 000, préférablement non supérieur à 500, plus préférablement non supérieur à 150, plus préférablement non supérieur à 100, plus préférablement non supérieur à 75.

9. Procédé selon les revendications 6 à 8, la composition comprenant d'environ 5 % à environ 99 %, et préférablement d'environ 5 % à environ 95 % en poids du HFCO-1233zd.
